# FASCICULE DE BREVET EUROPEEN

(11) **EP 0 389 333 B1**
(45) Date de publication et mention de la délivrance du brevet: **29.12.1993**
(21) Numéro de dépôt: 90400661.6
(22) Date de dépôt: 13.03.1990
(51) Int. Cl.: A61B 6/03

(54) **Procédé d'acquisition de données radiologiques relatives à un corps irradié et de reconstruction de structures correspondant à ce corps**
Verfahren zur Erfassung von radiologischen Daten und zur Rekonstruktion des Aufbaus eines bestrahlten Körpers
Process for the acquisition of radiological data and the reconstruction of structures of an irradiated body

(30) Priorité: 20.03.1989 FR 8903606
(43) Date de publication de la demande: 26.09.1990
(73) Titulaire: GENERAL ELECTRIC CGR S.A., F-92130 Issy les Moulineaux (FR)
(72) Inventeur: Saint Felix, Didier, Cabinet Ballot-Schmit, F-75116 Paris (FR); Trousset, Yves, Cabinet Ballot-Schmit, F-75116 Paris (FR); Picard, Catherine, Cabinet Ballot-Schmit, F-75116 Paris (FR); Rougee, Anne, Cabinet Ballot-Schmit, F-75116 Paris (FR)
(74) Mandataire: Schmit, Christian Norbert Marie

(56) Documents cités:
- FR-A- 2 368 058
- US-A- 4 128 877
- IEEE TRANSACTIONS ON NUCLEAR SCIENCE. vol. NS-25, no. 5, octobre 1978, New York,US; pages 1135 - 1143; SCHLINDWEIN: "Iterative three-dimensional reconstruction from twin-cone beam projections"

## Description

### Procédé d'acquisition de données radiologiques relatives à un corps irradié et de reconstruction de structures correspondant à ce corps.

La présente invention à pour objet un procédé d'acquisition de données radiologiques relatives à un corps irradié par des rayonnements X et de reconstruction de structures correspondant à ce corps. La reconstruction des structures vise à la représentation d'images de coupes, ou d'images de vues, de ces structures. Le procédé concerne essentiellement l'acquisition de données radiologiques détectées par un détecteur à deux dimensions placé en regard de l'émetteur de rayons X. Par opposition aux tomodensitomètres classiques, le rayonnement X irradie ici le corps en volume et l'absorption de ce rayonnement dans le corps peut être mesurée en même temps pour tout le volume du corps, et non plus seulement dans une tranche de ce volume. Le but des acquisitions de ce type est bien entendu d'accélérer la phase de mesure du phénomène d'absorption radiologique dans tout le corps.

On connaît ce mode d'acquisition, par exemple présenté au deuxième colloque image de la "Semaine Internationale de l'Image Electronique" à Nice en Avril 1986 par Pierre GRANGEAT. Par opposition à la tomodensitométrie, on parle alors de voludensitométrie, et un système de base permettant de reconstruire les structures étudiées est le suivant. Il comporte une source X ponctuelle, et le flux de rayonnement a une géométrie conique. Cette source est placée en regard d'un corps à examiner. De l'autre côté du corps par rapport à la source est placé, sensiblement orthogonalement à une direction principale du rayonnement, un détecteur bidimensionnel. Ce détecteur bidimensionnel est susceptible d'acquérir des données radiologiques qui peuvent être numérisées.

En faisant tourner l'ensemble d'acquisition, comprenant la source et le détecteur bidimensionnel, autour du corps on peut réitérer l'irradiation et disposer d'un ensemble d'acquisitions. Par un algorithme de reconstruction on est alors capable de répartir sur un quadrillage spatial des informations numériques représentatives d'une reconstruction de la structure du corps soumis à l'examen. Les algorithmes de reconstructions mis en oeuvre dans ce type d'investigation comportent essentiellement une utilisation de la transformé de Radon 3D et de sa formule d'inversion. Autrement dit, en réduisant à quelques secondes la phase d'acquisition avec un tel système, et en utilisant un algorithme de reconstruction adéquat, on aboutit au résultat recherché.

Les algorithmes de reconstruction ainsi évoqués nécessitent que la trajectoire de la source de rayon X, et en correspondance celle du détecteur 2D, soient circulaires par rapport au corps à examiner. Comme la géométrie conique du rayonnement empêche de décomposer le problème de reconstruction en une superposition de reconstructions bi-dimensionnelles suivant des coupes parallèles, perpendiculaires à l'axe de rotation de l'ensemble, on est conduit à développer des algorithmes fondés sur des calculs approchés de la transformée de Radon 3D et de son inversion.

L'utilisation d'approximations conduit à des artéfacts de reconstruction de sorte que lorsqu'on veut ainsi visualiser l'objet reconstruit, en y sélectionnant des coupes ou en le visualisant directement, on voit apparaître ces artéfacts dans les images présentées. Celles-ci ne sont alors pas facilement exploitables.

Dans une autre approche on effectue la reconstruction en simulant la structure reconstruite, en projetant mathématiquement la simulation de cette structure conformément à une direction d'irradiation, et en comparant l'image en projection simulée directement avec les mesures résultant de l'irradiation. En fait, on effectue cette comparaison non pas pour une seule direction d'irradiation mais pour toutes les directions d'irradiation effectuées réellement : à chaque direction d'acquisition correspond une projection de la structure simulée. De cette comparaison on déduit une modification du modèle simulé de la structure étudiée. Le modèle simulé modifié est alors, en une itération suivante, projeté comme précédemment et sa projection est à nouveau comparée aux acquisitions réellement faites. Ainsi de suite jusqu'à ce que les comparaisons montrent que le modèle simulé modifié est suffisamment proche de la structure réellement irradiée. Ces procédés de reconstructions par estimation et simulation mettant en oeuvre des algorithmes de reconstructions dits algébriques sont tout à fait adaptés lorsque le nombre d'acquisition est faible ou lorsque les irradiations sont mal réparties autour du corps. Le nombre est faible en particulier lorsqu'il s'agit d'angiographie pour lequel l'injection traumatisante d'un produit de contraste ne peut pas être répétée trop souvent. Elles sont mal réparties quand la géométrie du corps à étudier, ou les moyens d'intervention sur ce corps, ne permettent pas de disposer l'ensemble source-détecteur correctement sur tout le pourtour d'un cercle entourant la zone d'intérêt de l'objet.

L'invention a pour objet, à nombre d'acquisition égal, et en mettant en oeuvre un algorithme algébrique de reconstruction, de concourir à la reconstruction de structures avec moins d'artéfacts que dans l'état de la technique connu. Tout simplement dans l'invention, plutôt que de faire décrire a la source de rayons X (et en correspondance au détecteur 2D) une trajectoire circulaire, on préfère répartir les orientations des directions principales d'acquisition dans l'espace, de manière à ce que toutes ces orientations ne puissent pas être comprises dans un même plan. Le nombre d'acquisition sera au minimum de trois. De préférence, ces trois orientations des directions d'irradiations utilisées seront orthogonales entres elles. Le fait de ne pas avoir affaire à des orientations situées dans un même plan est associé avec l'utilisation d'un algorithme algébrique de reconstruction.

L'invention a donc pour objet un procédé d'acquisition de données radiologiques relatives à un corps irradié par des rayonnements X, et de reconstruction de structures correspondant à ce corps en vue de leurs représentations imagées, comprenant, pour acquérir, les étapes suivantes
- on émet un flux de rayons X vers le corps avec un émetteur, ce flux comportant une direction principale d'irradiation et ayant une géométrie conique pour permettre une irradiation en volume du corps à examiner;
- on détecte, avec un détecteur à deux dimensions maintenu vis à vis de l'émetteur, une vue relative à une orientation donnée de la direction principale par rapport au corps, cette vue consistant en une collection de données fonction des coordonnés de lieux sur ce détecteur et fonction de l'atténuation dans le corps de la partie des rayons X qui aboutissent en ces lieux,
- on réitère les étapes précédentes pour des directions principales ayant des orientations différentes par rapport au corps,
   et, pour reconstruire, les étapes suivantes
- on met en oeuvre un algorithme algébrique de reconstruction ;
   caractérisé en ce que, pour acquérir,
- on distribue ces orientations différentes de façon qu'un plan formé par deux directions principales, ou des directions parallèles à ces directions principales, coupe au moins une troisième direction principale d'irradiation.

L'invention sera mieux comprise à la lecture de la description qui suit et à l'examen des figures qui l'accompagnent. Celles-ci ne sont données qu'à titre indicatif et nullement limitatif de l'invention.

Les figures montrent :
- figures 1 : une représentation schématique de moyens utilisés pour mettre en oeuvre le procédé de l'invention ;
- figure 2a et 2b : une représentation schématique de la justification de l'invention ;
- figure 3 : un exemple de mise en oeuvre de l'invention dans un tomodensitomètre de type classique.

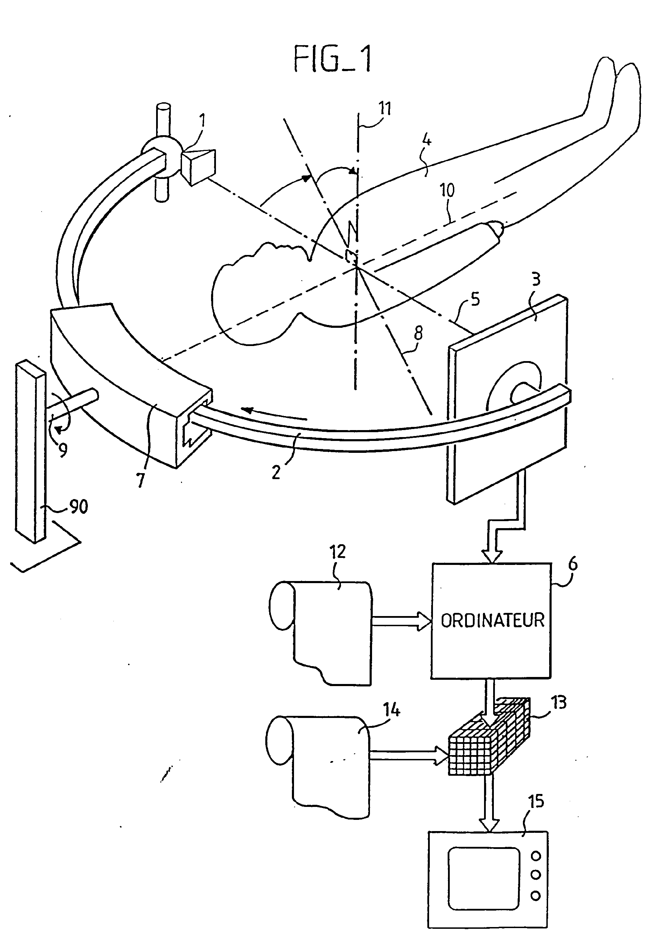

La figure 1 représente schématiquement et en perspective des moyens utilisables pour mette en oeuvre le procédé de l'invention. Ces moyens comportent un émetteur 1 de rayons X maintenu, par exemple par un arceau 2 en regard d'un détecteur 3 à deux dimensions. Le détecteur 3 est approximativement plan et possède en son centre une direction normale confondue avec une direction principale d'irradiation 5. Un corps 4 à examiner est soumis à une irradiation dont la direction principale 5 d'irradiation est déterminée par rapport à la position du corps 4. Par exemple quand l'examen radiologique est un examen cardiaque cette orientation peut être latérale, de la gauche vers la droite du patient, et être orienté légèrement en biais, de la tête vers les pieds du patient. Le signal délivré par le détecteur 3 est traité dans un ordinateur 6. Il est notamment numérisé de manière à ce que à chaque irradiation corresponde une collection de données radiologiques dont les valeurs sont fonction du lieu sur le détecteur d'où la mesure provient (ces données concernent l'adresse sur le détecteur du lieu considéré), et qui est aussi fonction de l'atténuation dans le corps 4 de la partie du rayonnement X qui a abouti en ce lieu.

Ces résultat étant obtenus, on fait tourner l'ensemble source 1-détecteur 3 autour du patient par exemple en faisant coulisser l'arceau 2 dans un guide 7 de maintien. Dans ces conditions la direction principale d'irradiation 5 rejoint une nouvelle orientation 8 par rapport au corps 4. L'orientation 8 est dans les mêmes conditions que précédemment de la gauche vers la droite mais des pieds du patient vers la tête maintenant. A ce moment on réitère l'étape d'irradiation-détection précédente. En pratique on peut aussi imaginer que l'émission est continue mais que la lecture du détecteur n'est faite seulement que quand l'ensemble a pris les orientations 5 et 8.

Après que l'ensemble source 1-détecteur 3 a rejoint l'orientation 8 et qu'on y a acquis les mesures correspondantes, on fait basculer cet ensemble autour d'un pivot 9 d'axe horizontal 10, de manière à ce que l'orientation de la direction principale d'irradiation soit maintenant une orientation 11 orientée sensiblement orthogonalement au plan formé par les deux orientations 5 et 8 précédentes. Dans ce but, le pivot 9 est fixé au guide 7 et à un piédestal 90. On acquiert, quand l'ensemble source-détecteur est dans cette orientation 11 une radiographie frontale.

Les données radiologiques acquises lorsque l'ensemble avait les orientations 5, 8 et 11 sont stockées dans la mémoire de l'ordinateur. Un programme de reconstruction 12 mettant en oeuvre un algorithme algébrique de reconstruction est alors utilisé. Le résultat de l'utilisation de cet algorithme de reconstruction est matérialisé par la connaissance de la structure 13 reconstruite et correspondant au corps 3. Ces structures comportent un ensemble de données de densité radiologiques réparties sur un quadrillage spatial à trois dimensions.

Par des procédés de visualisation 14, de type connu, il est possible d'extraire de la structure 13 des images de coupes dans le corps 4 ou de visualisation des structures que ce corps contient. Ces images sont d'une manière connue représentées sur un moniteur de télévision 15 ou sur tout autre moyen de représentation.

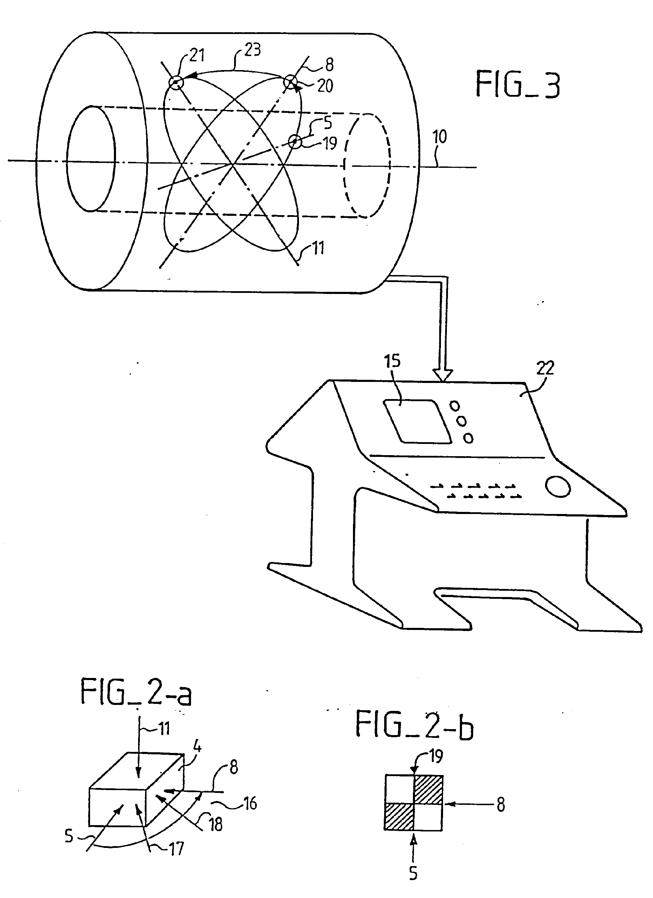

Les figures 2a et 2b illustrent l'intérêt d'utiliser le procédé d'acquisition selon l'invention. Si on utilise les explorations de type classique d'une structure 4 à reconstruire les orientations des directions principales d'irradiations sont réparties sur une surface 16 qui les contient toutes. Par exemple la surface 16 comprend les directions 5, 17, 18 et 8. On comprend que si ces directions ne sont contenues que dans un plan 16, elles peuvent conduire à des indéterminations sur la position de certains détails à l'intérieur du corps 4, surtout si le nombre d'irradiations est faible.

Par contre ces indéterminations peuvent être franchement levées si on acquiert au moins une fois des données radiologiques alors que l'ensemble source-détecteur est selon une direction 11 orthogonale au plan 16.

Par exemple, figure 2b, quelque soit l'orientation d'irradiation 5 ou 8, le détecteur placé en regard de la structure 19 irradiée sera confronté à une ambiguïté de localisation des lieux à forte densité d'absorption radiologique par rapport aux autres à faible densité. En effet dans les deux cas (5,et 8) les résultats de détection sont comparables et ne permettent pas de discriminer les lieux de la structure 19 ou la densité est faible. En pratique ces indéterminations sont en partie levées en prenant un nombre important de mesures, ce qui n'est pas possible dans tous les cas comme on l'a vus précédemment. La levée d'ambiguïté est partielle car elle repose toujours sur la valeur de l'écart angulaire qui sépare les orientations. Plus cet écart est grand meilleur est la discrimination. Dans l'invention, tout en gardant un nombre restreint d'irradiations, on arrive à réduire simplement les ambiguïtés en orientant une des irradiations (11) au moins, sensiblement orthogonalement au plan 16 formé par la répartition des autres.

La figure 3 montre un tomodensitomètre de type classique modifié pour pouvoir mettre en oeuvre l'invention. La modification consiste d'abord à remplacer le détecteur à une dimension de cet appareil par un détecteur de type 2D. Ce détecteur 2D peut être un détecteur au silicium ou éventuellement un écran intensificateur de luminance.

Tous les tomodensitomètres modernes permettent d'obtenir des images de coupes obliques. Dans ce but, une couronne verticale qui contient l'ensemble source-détecteur susceptible de tourner autour du patient pendant l'acquisition des différentes vues, peut être basculée vers l'avant ou vers l'arrière de quelque degrés. Pour mettre en oeuvre l'invention avec un tel appareil il suffit par exemple d'acquérir deux vues alors que la couronne est basculé vers l'arrière. Ces deux vues y ont des orientations 5 et 8, et pour ces deux vues une source de rayons X serait pas exemple dans les positions respectivement 19 et 20. Une fois ces deux vues acquises il suffit de basculer la couronne vers l'avant et d'acquérir une troisième vue selon une direction 11 alors que la source de rayon X est dans la position 21. En passant de quinze degré vers l'arrière à quinze degré vers l'avant, le basculement est de l'ordre de 30°. Il permet déjà d'explorer sensiblement "orthogonalement" la structure à examiner et si celle-ci est placée colinéairement à l'axe 10 de la machine.

La console 22 d'une telle machine comprend tous les moyens d'ordinateur et de mémoire pour mettre en oeuvre les programmes 12 et 14 de reconstruction et de visualisation des structures reconstruites sur l'écran 15.

Un algorithme algébrique de reconstruction de type itératif à été pour la première fois décrit dans "Images reconstructions from projections" G.T Herman Academic Press 1980. Un perfectionnement à cet algorithme a par ailleurs été proposé par K.M Hanson et G. W Wecksung dans "LOCAL BASIS - FUNCTIONS APPROACH TO COMPUTED TOMOGRAPHY", Applies Optics, Vol 24 N° 23, December 1985 pages 4028-4039. L'application de ces algorithmes aux données radiologiques acquises selon l'invention est immédiate puisqu'on connaît à chaque fois la géométrie de l'ensemble de projection-irradiation.

## Revendications

1. Procédé d'acquisition de données radiologiques relatives à un corps (4) irradié (1) par des rayonnements X, et de reconstruction de structures correspondant à ce corps en vue de leurs représentations (15) imagées, comprenant, pour acquérir, les étapes suivantes
- on émet un flux de rayons X vers le corps avec un émetteur (1), ce flux comportant une direction (5) principale d'irradiation et ayant une géométrie conique pour permettre une irradiation en volume du corps à examiner ;
- on détecte, avec un détecteur (3) à deux dimensions maintenu (2,7) vis à vis de l'émetteur, une vue relative à une orientation donnée de la direction principale par rapport au corps, cette vue consistant en une collection de données fonction des coordonnés de lieux sur ce détecteur et fonction de l'atténuation dans le corps de la partie des rayons X qui aboutissent en ces lieux,
- on réitère les étapes précédentes pour des directions principales ayant des orientations (8) différentes par rapport au corps,
et, pour reconstruire, l'étape suivante
- on met en oeuvre un algorithme algébrique de reconstruction ;
caractérisé en ce que, pour acquérir,
- on distribue ces orientations différentes de façon qu'un plan (16) formé par deux directions principales (58), ou des directions parallèles à ces directions principales, coupe au moins une troisième (11) direction principale d'irradiation.

## Patentansprüche

1. Verfahren zur Erfassung von sich auf einen durch Röntgenstrahlen (X) bestrahlten (1) Körper (4) beziehenden radiologischen Daten und zur Rekonstruktion von Strukturen entsprechend dem Körper im Hinblick auf deren Bilddarstellungen (15), aufweisend zum Erfassen die folgenden Schritte
- es wird ein Fluß von Röntgenstrahlen zum Körper mit einem Sender (1) ausgesendet, wobei der Fluß eine Hauptbestrahlungsrichtung (5) umfaßt und eine konische Geometrie aufweist, um eine Volumenbestrahlung des zu untersuchenden Körpers zu gestatten;
- es wird mit einem gegenüber dem Sender gehaltenen (2, 7) Flächendetektor (3) eine Ansicht bezüglich einer gegebenen Orientierung der Hauptrichtung in bezug auf den Körper detektiert, wobei die Ansicht aus einer Datenmenge besteht, abhängig von den Ortskoordinaten auf dem Detektor und abhängig von der Abschwächung des Teils der Rontgenstrahlen im Körper, die zu diesen Orten hinführen;
- es werden die vorhergehenden Schritte für Hauptrichtungen wiederholt, die verschiedene Orientierungen (8) in bezug auf den Körper aufweisen;
und zum Rekonstruieren den folgenden Schritt
- es wird ein algebraischer Rekonstruktionsalgorithmus ausgeführt;
dadurch **gekennzeichnet**, daß zum Erfassen
- die verschiedenen Orientierungen so verteilt werden, daß eine durch zwei Hauptrichtungen (58) oder zu den Hauptrichtungen parallele Richtungen gebildete Ebene (16) wenigstens eine dritte (11) Hauptbestrahlungsrichtung schneidet.

## Claims

1. Method for acquiring radiological data relating to a body (4) irradiated (1) by X-rays, and for reconstructing structures corresponding to this body for the purpose of their representation (15) as images, comprising, for the purpose of acquisition, the following steps:
- a flow of X-rays is emitted towards the body with an emitter (1), this flow having a principal irradiation direction (5) and having a conical geometry so as to allow a volume irradiation of the body to be examined;
- with a two-dimensional detector (3) held in position (2, 7) facing the emitter, a view is detected relating to a given orientation of the principal direction with respect to the body, this view consisting of a collection of data as a function of the coordinates of places on this detector and as a function of the attenuation in the body of the part of the X-rays which reach these places,
- the preceding steps are repeated for principal directions with different orientations (8) with respect to the body,
and, for the purpose of reconstruction, the following step:
- an algebraic reconstruction algorithm is used;
characterised in that, for the purpose of acquisition,
- these different orientations are distributed so that a plane (16) formed by two principal directions (58), or directions parallel to these principal directions, intercepts at least a third (11) principal irradiation direction.
